Europäisches Patentamt

European Patent Office

Office européen des brevets

(11) Veröffentlichungsnummer: **0 296 541**
**A1**

(12) **EUROPÄISCHE PATENTANMELDUNG**

(21) Anmeldenummer: **88109872.7**

(22) Anmeldetag: **21.06.88**

(51) Int. Cl.⁴: **G01N 19/00 , G01N 33/18**

(30) Priorität: **24.06.87 DE 3720807**

(43) Veröffentlichungstag der Anmeldung:
**28.12.88 Patentblatt 88/52**

(84) Benannte Vertragsstaaten:
**AT BE CH FR GB IT LI NL SE**

(71) Anmelder: **Seever, Wieland**
**Schaftrift 19**
**D-3100 Celle(DE)**

(72) Erfinder: **Seever, Wieland**
**Schaftrift 19**
**D-3100 Celle(DE)**

(74) Vertreter: **Patentanwälte Kohler - Schwindling**
**- Späth.**
**Hohentwielstrasse 41**
**D-7000 Stuttgart 1(DE)**

(54) **Messwertaufnehmer für Öle, Ölfraktionen Ölderivate und deren gasförmige Phase.**

(57) Ein Weßwertaufnehmer für Öle, Ölfraktionen, Ölderivate und deren gasförmige Phase, mit dem deren Vorhandensein, auch auf Wasser, signalisiert werden kann, und mit dem Ölgemische hinsichtlich deren Zusammensetzung beurteilt werden können, hat als Sensor (1) eine gespannte Polyäthylenfolie, die sich durch die genannten Fluide ausdehnt und nach deren Auswaschung wieder auf das ursprüngliche Maß verkürzt. Diese Wege werden von einem Meßwertwandler aufgenommen und in einer Meßkette verarbeitet.

Fig. 1

EP 0 296 541 A1

## Meßwertaufnehmer für Öle, Ölfraktionen, Ölderivate und deren gasförmige Phase

Die Erfindung betrifft einen Aufnehmer gemäß dem Oberbegriff des Anspruchs 1. Seine Anwendungsmöglichkeiten sind äußerst vielfältig. Er kann dazu dienen, ölführende Anlagen zu überwachen, um ein Auslaufen von Ölen zu signalisieren. Dabei kann er auch auf Wasseroberflächen Einsatz finden, um auch dünnste Ölfilme zu melden. Da Wasser bereits bei 1 p.p.M. Öl als verseucht gilt, ist eine äußerst hohe Ansprechempfindlichkeit für den Gewässerschutz notwendig. Der erfindungsgemäße Aufnehmer spricht auch auf Ölfilme auf Wasser in einer dünnstmöglichen Schichtdicke an.

Weiter können mit dem Aufnehmer Ölgemische und Ölfraktionen aus charakteristischen Zeit - Dehnungskurven identifiziert werden, so daß es auch möglich ist, mit Hilfe von Vergleichswerten Verursacher zu überführen.

Ein weiteres Anwendungsgebiet wäre die Qualitätsbeurteilung von Rohöl hinsichtlich seines prozentualen Anteils an Benzin.

Da der Aufnehmer auch auf die gasförmige Phase von leichtflüchtigen Ölfraktionen reagiert, ist es möglich, mit ihm auch Räume zu überwachen, um eine Explosionsgefährdung zu melden.

Die Reversibilität bzw. Regenerierbarkeit durch Auswaschen mit Reinbenzin oder anderen Lösungsmitteln macht ihn testbar und zu beliebig oft wiederholbarem Einsatz fähig.

Die bekannten technisch naheliegenden Systeme haben diese entscheidenden und zwingend nötigen Vorteile der Regenerierbarkeit und hohen Empfindlichkeit nicht. Zudem ist es mit ihnen nicht möglich, Öle und Ölfraktionen zu identifizieren. Außerdem ist ihr Anwendungsgebiet jeweils beschränkt auf Wasseroberflächen, Lufträumen oder Böden unter Öltanks.

Die meisten Systeme haben als Sensor einen in Öl unbeständigen und irreversibel auflösenden oder quellenden Kunststoff.( DT OS 23 65 435, DE OS 26 01 410, DE OS 25 29 233, DT OS 23 65 167, DE OS 31 40 804, DT OS 24 34 914, DT AS 23 44 778, DE AS 22 51 952, DT OS 24 53 215). Diese Systeme können nicht wiederholt getestet werden, da ihre Sensoren bereits nach dem ersten Einsatz zerstört werden. Zudem haben sie eine zu geringe Empfindlichkeit und zu große Trägheit, so daß sie für Alarmzwecke gerade für den Gewässerschutz untauglich sind. Außerdem sind sie teilweise nicht funktionssicher, da die durch Öleinwirkung freiwerdenden elektrischen Kontakte gerade durch das Öl wieder isoliert werden.

Andere Systeme ( DE AS 23 65 434, DE OS 25 05 596) verwenden Materialien, die hydrophob aber oleophil sind, und die durch anströmendes Öl eine Änderung des spezifischen oder absoluten Gewichtes erfahren und diese Änderung zur Betätigung eines Schalters führt. Auch hier sind die geringe Empfindlichkeit und große Trägheit die entscheidenden Nachteile. Über eine Reversibilität des Materials wird zudem nichts ausgesagt.

Bei der DE OS 25 28 026 und bei der DE OS 25 13 223 handelt es sich um Vorrichtungen, bei denen das Öl erst Schichten von hydrophoben aber oleophilen Filtern passiert, wobei das Wasser zurückgehalten und das durchgelassene Öl Änderungen von elektrischen Parametern bewirkt. Auch hier wird über eine Reversibilität nichts ausgesagt. Da die Lösungsmittel die oleophilen Filterschichten in beiden Richtungen erst passieren müßten, erscheint eine mögliche Reversibilität bzw. Regenerierbarkeit zumindestens langwierig wenn nicht unmöglich. Es ist auch wahrscheinlich, daß sich die Widerstandsschicht bei der DE OS 2528 026 durch die Öleinwirkung irreversibel ändert.

In der DE OS 27 56 872 wird eine Einrichtung beschrieben, bei der die unterschiedliche Wärmeübertragungscharakteristik zweier Flüssigkeiten wie z.B. Öl und Wasser mit Hilfe eines aufgeheizten Kaltleiters, der periodisch in die Flüssigkeitsschichten taucht, gemessen wird. Diese Einrichtung ist zwar reversibel, benötigt aber unvertretbar große Mengen der festzustellenden Flüssigkeit und ist damit für den Gewässerschutz ungeeignet. Zudem wird ein hoher apparativer Aufwand benötigt.

Ferner gibt es noch zahlreiche optische Systeme im ultravioletten und infraroten Bereich, oder Systeme, die Laserreflexionen nutzen, auf die hier nicht näher eingegangen wird, da auch bei ihnen der apparative Aufwand im Verhältnis zu dem im folgendem beschriebenen Aufnehmer unvergleichlich höher ist. Außerdem benötigen sie einen hohen Wartungsaufwand wegen der zu erwartenden Verschmutzung der optischen Systeme.

Der Erfindung liegt die Aufgabe zugrunde, zur Beseitigung der geschilderten Nachteile einen im Aufbau einfachen, testbaren, zuverlässigen und höchst sensiblen Aufnehmer zu schaffen, der zugleich ein breites Anwendungsspektrum hat.

Dieses Ziel erreicht die Erfindung bei dem im Oberbegriff des Anspruchs 1 genannten Aufnehmer dadurch, daß der Sensor aus einer gespannten Folie besteht, die einerseits gegen Öle, Ölfraktionen und Ölderivaten chemisch beständig ist, andererseits aber oleophil ist und sich in Anwesenheit der genannten-

2

Fluide auch in geringsten Mengen dehnt und sich nach deren Auswaschen wieder auf das ursprüngliche Maß verkürzt. Die neuartige Verwendung eines Materials, vorzugsweise Polyäthlen, mit voll reversiblem und zugleich auf unterschiedliche Ölgemische, Fraktio nen und Derivaten spezifischen Dehnungsverhalten gestattet einen verblüffend einfachen Aufbau des Aufnehmers. Zweckmäßig wird das Material in Form einer Folie vorgesehen, die umso schneller und sensibler reagiert, je dünner sie ist. Dadurch, daß das Material chemisch äußerst beständig ist und zugleich die verblüffende Eigenschaft der Reversibilität besitzt, ist der Aufnehmer zum wiederholten Einsatz fähig. Das spezifische Dehnungsverhalten auch auf so inerte Stoffe wie Paraffin oder Schmieröle gestattet zudem eine vielseitige Anwendung in der Alarmtechnik, wobei nicht nur die Anwesenheit eines zu erwartenden Stoffes sondern vieler umweltgefährdender Stoffe signalisiert werden kann, wobei zugleich durch die spezifische Reaktion, Verursacher überführt werden können.

Auch kann der Aufnehmer in der Analysetechnik Verwendung finden, um z.B. schnell die Qualität von Rohöl hinsichtlich des Benzingehaltes zu ermitteln.

Nachfolgend wird die Erfindung an Hand der in der Zeichnung dargestellten Ausführungsbeispiele zum besseren Verständnis näher erläutert.

Es zeigen:

Fig. 1 Einen Aufnehmer wahlweise ausgestattet mit einem Dehnungsmeßstreifen 5 oder einem Meßwertwandler 8.

Fig. 2 Einen Sensor 1,in dem ein Kondensator 14 integriert ist.

Fig. 3 Einen Sensor 1, in dem ein Kondensator oder ein anderer Meßwertwandler integriert ist.

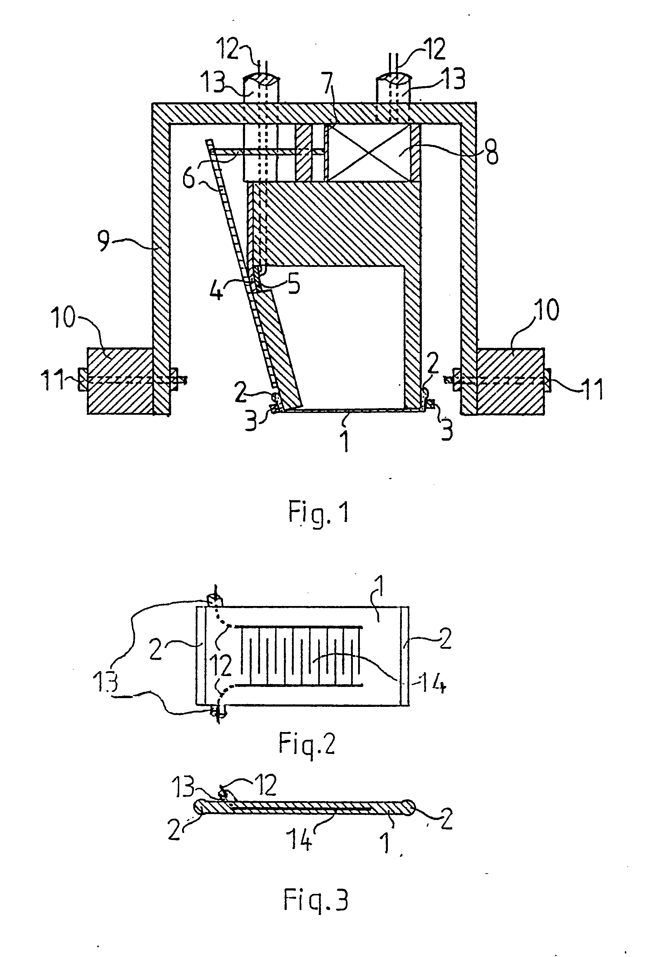

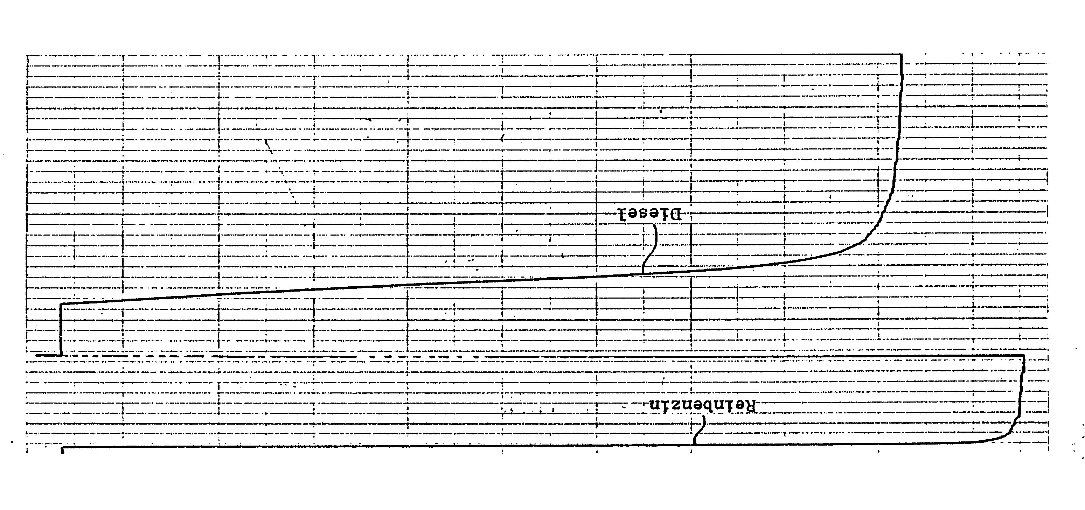

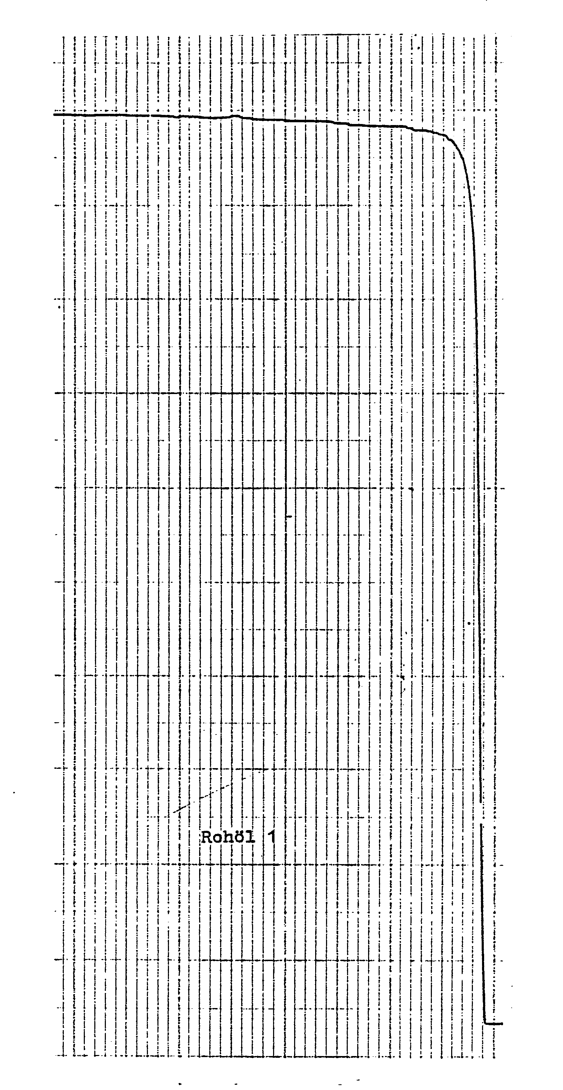

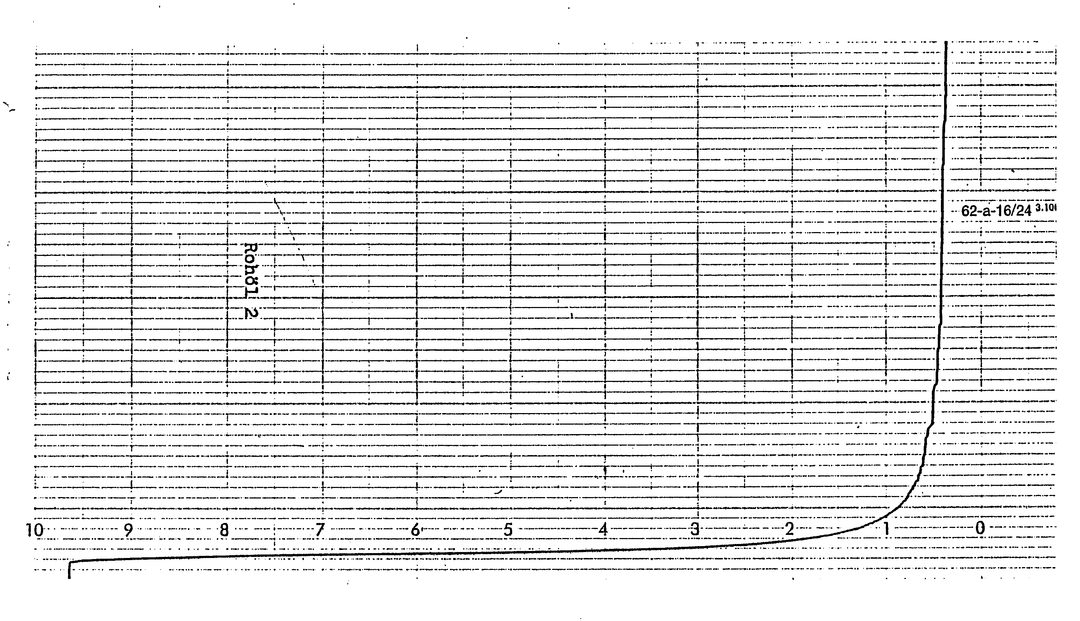

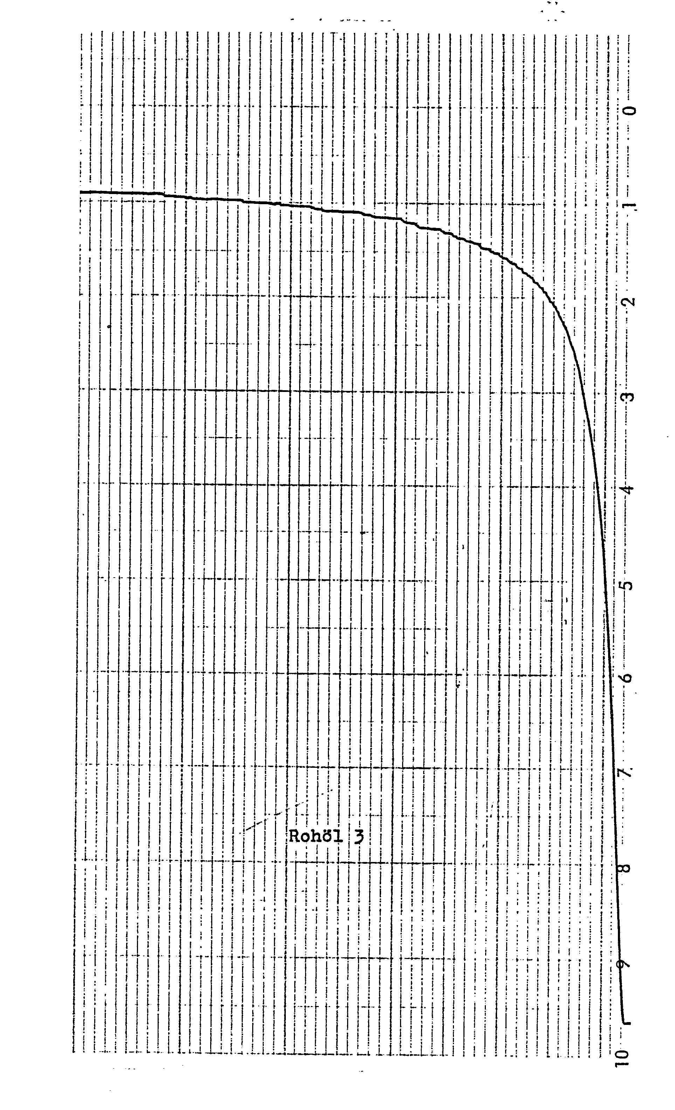

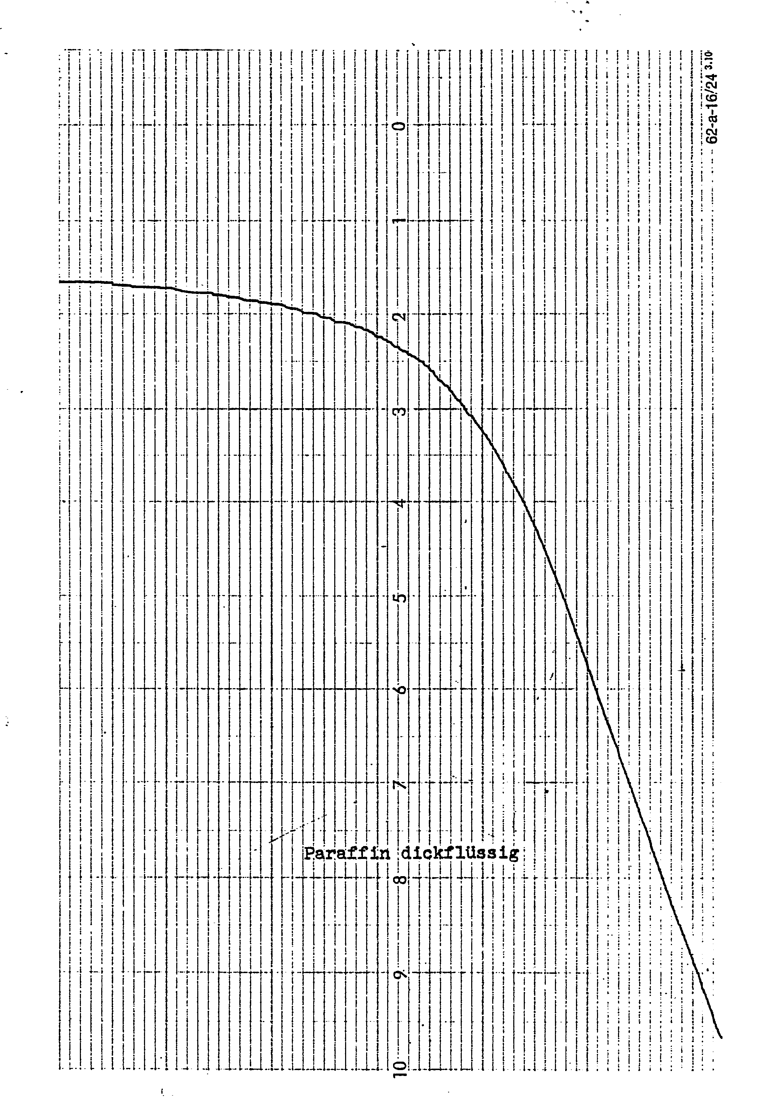

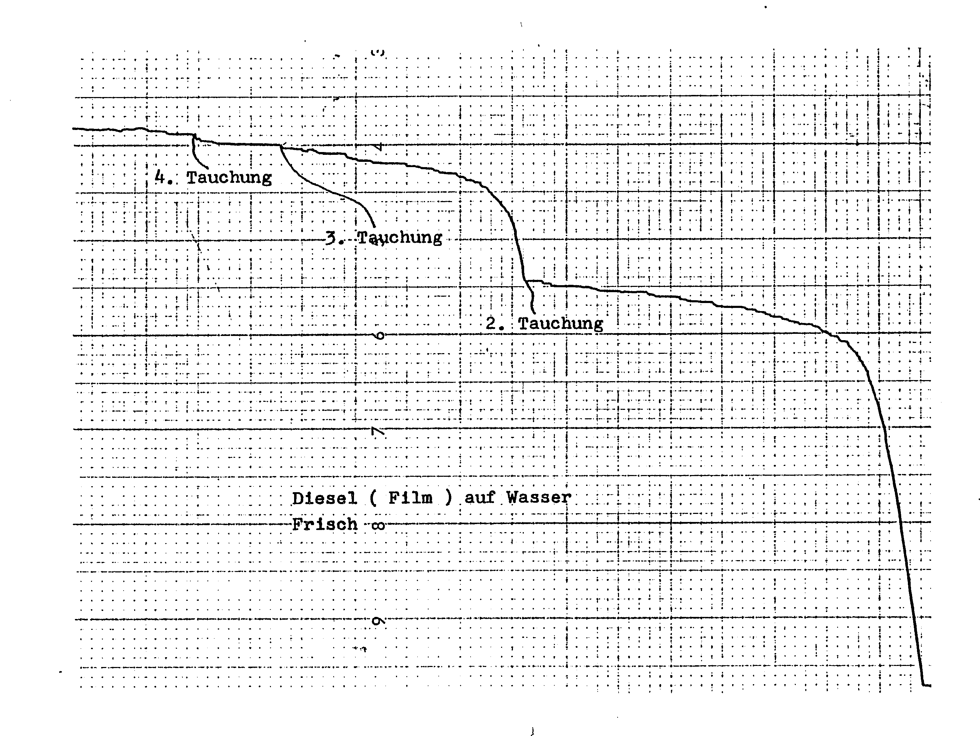

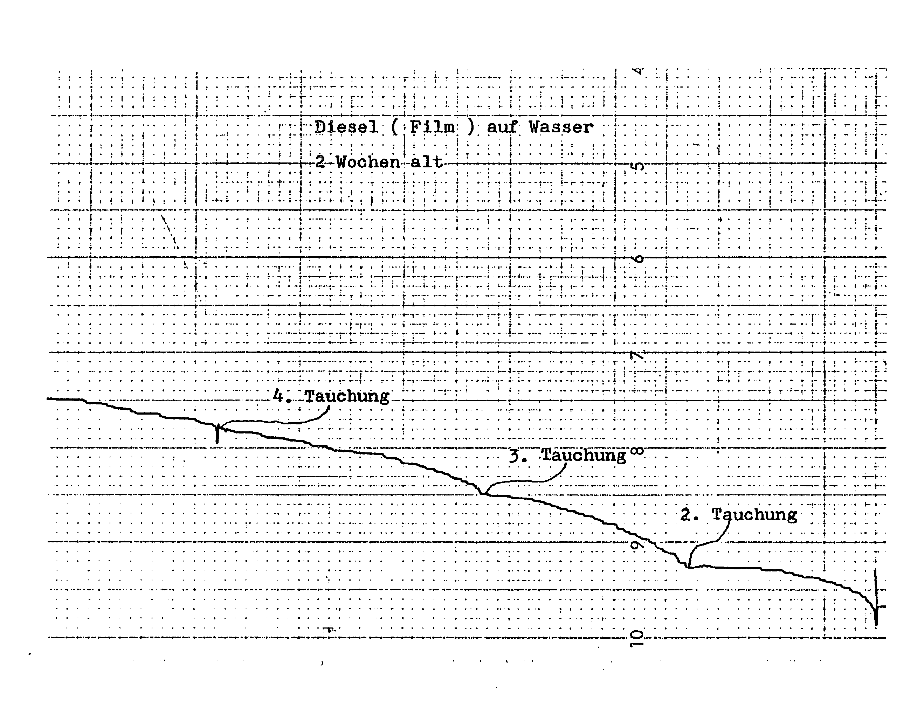

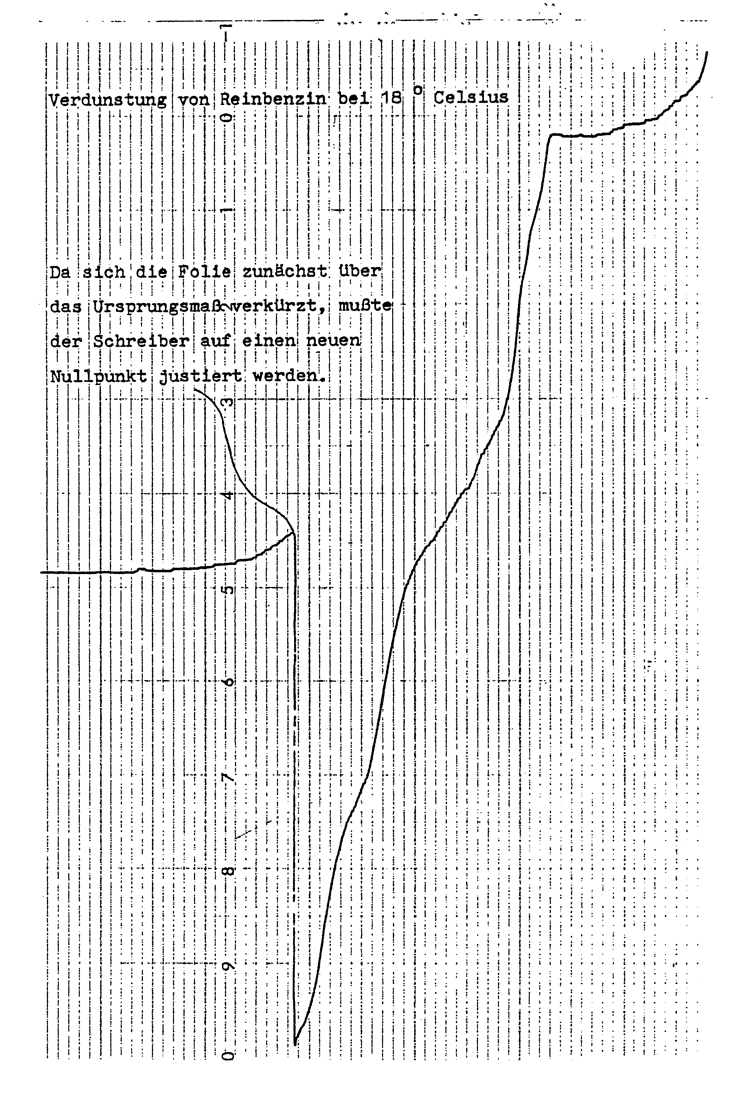

Fig. 1 zeigt ein nach unten offenes Gehäuse 9, an dem Schwimmer oder Füße 10 mit einer Schraube 11 verstellbar, z.B. in einem Langloch, befestigt sind. Mit Hilfe der Schwimmer oder Füße kann der Sensor 1 auf eine gewünschte Höhe oder auch Eintauchwinkel justiert werden. Nicht dargestellt sind die Löcher gemäß Anspruch 7 in den Seitenwandungen. Sie sind vorgesehen für einen Einsatz auf Wasseroberflächen, um Wasser durchströmen zu lassen, Fremdkörper aber, die bei starker Strömung den Sensor zerstören könnten, fernzuhalten.

Der Sensor ist an beiden Seiten mit Hilfe des Wulstes 2 mit den Halterungen 3 fixiert. Ist der durch die Halterungen gebildete Spalt an einem Ende offen, kann der Sensor bequem und schnell, etwa zum Regenerieren, ausgewechselt werden.

Die Biegefeder 4 hält den Sensor in der gewünschten Spannung. Durch den Dehnungsmeßstreifen 5, verbunden der elektrischen Zuführung 12 mit Isolierung 13, wird der Dehnungsweg aufgenommen, den der Sensor beim Kontakt mit dem entsprechenden Fluid macht.

Eine andere Möglichkeit besteht darin, den Dehnungsweg durch ein Hebelsystem 6 und über eine Membran 7, die den elektrischen Bereich vor Umwelteinflüssen schützt, auf einen anderen Aufnehmer zu übertragen. Die einfachste Ausführung bestände dabei in einem Schalter mit dem Signal 0 oder 1. Diese Ausführung wäre z.B. geeignet für Anwendungen bei Öl - führenden Anlagen, um ein Leck zu signalisieren. Dafür wäre der gesamte Aufnehmer so mit den Füßen zu justieren, daß der Sensor auf dem Boden unter einer Öl - führenden Anlage gerade aufläge.

Eine besonders vorteilhafte Ausgestaltung der Erfindung zeigen die Figuren 2 und 3.Innerhalb des Sensors ist ein Aufnehmer, z.B. ein Kondensator oder ein Dehnungsmeßstreifen 14 integriert. Der Sensor bildet also zugleich deren Trägermaterial.Die isolierte elektrische Zuleitung 12,13 führt direkt zu den Leitungsbahnen oder Schichten. Bei dieser Ausführung entfallen alle übriggen beweglichen Teile. Es genügt, den Sensor durch eine Feder unter Spannung zu halten. Die Leiterbahnen oder Schichten sind, dadurch daß sie vom Sensormaterial umgeben sind, zugleich vor Wasser und vor den zu messenden Fluiden geschützt.

Um eine noch höhere Empfindlichkeit bei allen Ausführungen für den Einsatz auf Wasseroberflächen zu erreichen, sollte der Sensor gemäß den Ansprüchen 4 - 6 periodisch durch Bewegung in die Wasseroberfläche getaucht werden. Da er aus oleophilem Material ist, sammelt er gleichsam immer mehr von dem zu messenden Fluid, so daß sich Mengen auf ihm summieren und zu einer gewünschten deutlichen Registrierung führen.

Bei allen Ausführungen sollte der Sensor zuvor unter Spannung ca. 20 mal in Reinbenzin getaucht werden. Zwischen den Tauchvorgängen muß das Reinbenzin wieder verdunsten, Es zeigt sich nämlich, daß während des Verdunstungsvorganges, also während der Verkürzung, die Folie zusätzlich bis zu einem Maximum irreversibel gestreckt wird. Außerdem sollte der Sensor ebenfalls unter Spannung mindestens mit einer zu erwartenden Höchsttemperatur im Einsatzgebiet getempert werden. Auch beim Tempern wird die Folie irreversibel gestreckt, und sie wird dadurch in ihrem Temperaturverhalten stabiler.

Bei einem Temperaturgang von 0° bis 40° Celsius verbleibt dann noch eine reversibele Dehnung von ca. 14 % der Gesamtdehnung durch Reinbenzin. Da ein klimatischer Temperaturgang und die damit verbundene Dehnung im Gegensatz zur Dehnung durch Fluide äußerst langsam ist und dadurch bei der Meßwerterfassung als solcher zu erkennen ist, ist eine weitere Temperaturkompensation für Alarmzwecke nicht notwendig. Ist der Sensor mit einem Schalter gekoppelt, genügt es, den Schalter mit einem entsprechend langem Schaltweg auszustatten, so daß ein Dehnungsweg durch einen Temperaturgang den Schalter nicht ansprechen läßt.

.Für höhere Ansprüche an die Meßgenauigkeit, etwa beim Bestimmen des Benzingehaltes von Rohöl, wäre eine weitere Temperaturkompensation nötig. Sie kann gemäß Anspruch 37 dadurch erfolgen, daß der gesamte Aufnehmer mit einem Temperaturaufnehmer, der mit in die zu messende Flüssigkeit taucht, versehen ist, und dessen Signale temperaturkompensatorisch in der Meßkette elektronisch verarbeitet werden. Eine weitere Möglichkeit der Temperaturkompensation bestände darin, den gesamten Ausnehmer gemäß den Ansprüchen 35 oder 36 mit einer zweiten eingespannten Folie, die dasselbe Temperaturverhalten wie die Sensorfolie hat, zu versehen, die aber entweder vor Öleinflüssen geschützt ist oder aus einem Material besteht, das nicht auf Öl reagiert. Durch eine dem Sensor gleiche elektrische Kopplung ist es dann möglich, die Temperaturdehnung z.B. in einer Meßbrücke nach bekanntem Verfahren zu kompensieren.

Die folgenden Kurven wurden mit einem Aufnehmer gewonnen, dessen Sensor aus einer ca. 0,008 mm starken Polyäthylenfolie mit den Maßen 1,5 X 1,5 cm bestand. Die Werte wurden mit zwei Dehnungsmeßstreifen auf einer Biegefeder aufgenommen. Die Zugkraft der Biegefeder betrug ca. 0,7 N. Die maximale Dehnung des Sensors in Reinbenzin betrug ca. 1,7 mm. Der Schreiber hatte einen Vollausschlag von 10 mV und einen Vortrieb von 152,4 cm pro Stunde. Die Meßbrücke wurde mit 6 V gespeist. Die Verstärkung war zwanzigfach. Die Dehnungsmeßstreifen hatten eine Impendanz von 120Ω.

Bei Ausführungsformen der Erfindung kann der Meßwertwandler eines der folgenden Elemente sein: ein Widerstandsaufnehmer, kapazitiver Aufnehmer, induktiver Aufnehmer, piezoelektrischer Aufnehmer, Saitendehnungsaufnehmer, optoelektronischer Aufnehmer, elektromagnetischer Aufnehmer, Feldplattenaufnehmer, eine Hallsonde, ein Reed-Kontakt oder ein Schalter.

Der Meßwertaufnehmer kann erfindungsgemäß mit einer Meßkette gekoppelt sein. Der Meßwertaufnehmer kann erfindungsgemäß auch mit einem Sender gekoppelt sein.


**Ansprüche**

1. Meßwertaufnehmer für Öle, Ölfraktionen,Ölderivate und deren gasförmige Phase, dadurch gekennzeichnet, daß eine unter Spannung gehaltene Folie (1) als Sensor sich in Anwesenheit des entsprechenden Fluids oder dessen gasförmige Phase spezifisch ausdehnt und bei dessen Abwesenheit wieder auf das ursprüngliche Maß verkürzt, und diese Wege auf einen Meßwertwandler übertragen werden.

2. Meßwertaufnehmer nach Anspruch 1, dadurch gekennzeichnet, daß er mit einem nach einer Seite offenem Gehäuse versehen ist.

3. Meßwertaufnehmer nach Anspruch 2, dadurch gekennzeichnet, daß das Gehäuse mit verstellbaren Füßen oder Schwimmern (10) versehen ist.

4. Meßwertaufnehmer nach einem der vorhergehenden Ansprüche 1 - 3, dadurch gekennzeichnet, daß der Meßwertaufnehmer mittels einer Antriebsvorrichtung in Bewegung versetzt wird.

5. Meßwertaufnehmer nach einem der vorhergehenden Ansprüche 1 - 4, dadurch gekennzeichnet, daß der Sensor (1) mittels einer Antriebsvorrichtung in eine zum Gehäuse (9) relativen Bewegung versetzt wird.

6. Meßwertaufnehmer nach einem der Ansprüche 1 - 5, dadurch gekennzeichnet, daß die Wasseroberfläche auf der er schwimmt, mittels eines mit einer Antriebsvorrichtung gekoppelten Stempels in Bewegung versetzt wird.

7. Meßwertaufnehmer nach einem der vorhergehenden Ansprüche 1 - 6, dadurch gekennzeichnet, daß die Wandungen des Gehäuses Öffnungen haben.

8. Meßwertaufnehmer nach Anspruch 1, dadurch gekennzeichnet, daß der Sensor mittels einer Biegefeder (4) unter Spannung gehalten wird.

9. Meßwertaufnehmer nach Anspruch 1 und 8, dadurch gekennzeichnet, daß der Sensor an den eingespannten Enden einen Wulst (2) hat, der dicker ist als der durch die Halterung (3) gebildete Spalt, und daß der Spalt nach einer Seite offen ist.

10. Maßwertaufnehmer nach einem der vorhergehenden Anspüche 1 - 9, dadurch gekennzeichnet, daß der Sensor unter Spannung getempert ist.

11. Meßwertaufnehmer nach Anspruch 10, dadurch gekennzeichnet, daß der Sensor vor Inbetriebnahme mehrfach in Reinbenzin getaucht ist, und zwischenzeitlich das Rohbenzin verdunstet ist.

12. Meßwertaufnehmer nach Anspruch 10 und 11, dadurch gekennzeichnet, daß der Sensor U V - stabilisiert ist.

13. Meßwertaufnehmer nach den.Ansprüchen 10 - 12, dadurch gekennzeichnet, daß der Sensor mit einem Antialgenmittel behandelt ist.

14. Meßwertaufnehmer nach einem der vorhergehenden Ansprüche 1 - 13,dadurch gekennzeichnet, daß der Sensor aus einer Kunststoffolie, vorzugsweise Polyäthylen, besteht.

15. Meßwertaufnehmer nach einem der vorhergehenden Ansprüche 1 -14, dadurch gekennzeichnet, daß der Sensor mit einer oder mehreren leitenden Schichten oder Bahnen versehen ist.

16. Meßwertaufnehmer nach Anspruch 15,dadurch gekennzeichnet, daß Bahnen oder Schichten vom Sensormaterial umschlossen sind.

17. Meßwertaufnehmer nach Anspruch 15 oder 16, dadurch gekennzeichnet,daß die Bahnen oder Schichten (14) einen Kondensator bilden.

18. Meßwertaufnehmer nach Anspruch 15 oder 16, dadurch gekennzeichnet, daß die Bahnen oder Schichten (14) einen Widerstand bilden.

19. Meßwertaufnehmer nach Anspruch 15 oder 16, dadurch gekennzeichnet, daß die Bahnen oder Schichten (14) aus Halbleitermaterial sind.

20. Meßwertaufnehmer nach den Ansprüchen 15 - 19, dadurch gekennzeichnet, daß die Bahnen oder Schichten (14) mit einer elektrischen Zuführung (12) mit einer Isolierschicht (13) versehen sind.

21. Meßwertaufnehmer nach einem der vorhergehenden Ansprüche 1 - 14, dadurch gekennzeichnet, daß die Biegefeder (4) mit einem oder mehreren Dehnungsmeßstreifen (5) versehen ist.

22. Meßwertaufnehmer nach einem der vorhergehenden Ansprüche 1 - 14, dadurch gekennzeichnet, daß der Sensor (1) durch ein Hebelsystem (6) über eine Membran (7) mit einem Meßwertwandler (8) gekoppelt ist.

23. Meßwertaufnehmer nach einem der vorhergehenden Ansprüch 1 - 14, dadurch gekennzeichnet, daß der Sensor (1) durch eine hydraulische Vorrichtung mit einem Meßwertwandler (8) gekoppelt ist.

24. Meßwertaufnehmer nach einem der vorhergehenden Ansprüche, dadurch gekennzeichnet, daß er mit einer zweiten mit einem ölundurchlässigem Überzug versehenen Folie gleichen Sensormaterials, die mit einem jeweils verwendeten gleichem Aufnehmer gekoppelt ist, oder die die gleichen Bahnen oder Schichten wie der Sensor hat, versehen ist.

25. Meßwertaufnehmer nach Anspruch 24, dadurch gekennzeichnet, daß die zweite Folie aus einem nicht ölansprechendem Material ist, aber die gleiche Wärmeausdehnung hat.

26. Meßwertaufnehmer nach einem der Ansprüche 1 - 23, dadurch gekennzeichnet, daß er mit einem Temperaturaufnehmer versehen ist.

Fig. 1

Fig.2

Fig.3

Rohöl 1

62-a-16/24 3.10

Rohöl 2

Rohöl 3

Paraffin dickflüssig

62-a-16/24 3.10

2. Tauchung

3. Tauchung

4. Tauchung

Diesel ( Film ) auf Wasser

Frisch

Diesel (Film) auf Wasser

2 Wochen alt

4. Tauchung

3. Tauchung

2. Tauchung

Verdunstung von Reinbenzin bei 18 ° Celsius

Da sich die Folie zunächst über
das Ursprungsmaß verkürzt, mußte
der Schreiber auf einen neuen
Nullpunkt justiert werden.

Europäisches
Patentamt

**EUROPÄISCHER RECHERCHENBERICHT**

Nummer der Anmeldung

EP 88 10 9872

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-4 058 802 (F. MEYERS) <br> * Spalte 4, Zeile 36 - Spalte 5, Zeile 68; Spalte 9, Zeile 39 - Spalte 10, Zeile 9; Figuren 2,3 * <br> --- | 1,7,14 | G 01 N 19/00 <br> G 01 N 33/18 |
| Y | US-A-3 674 439 (L.E. HAKKA et al.) <br> * Spalte 1, Zeilen 30-40; Spalte 2, Zeilen 17-31,39-57; Figuren 1,2 * <br> --- | 1,7,14 | |
| A | US-A-3 719 936 (R.H. DANIELS et al.) <br> * Spalte 5, Zeilen 2-10; Figur 5 * <br> --- | 3,7 | |
| A | PATENT ABSTRACTS OF JAPAN, Band 8, Nr. 65 (P-263)[1502], 27. März 1984; & JP-A-58 213 232 (MEISEI DENKI K.K.) 12-12-1983 <br> --- | 8,21 | |
| A | FR-A-2 455 294 (INSTITUT FRANCAIS DE PETROLE) <br> * Seite 14, Zeilen 1-15 * <br> --- | 12 | |
| A | PATENT ABSTRACTS OF JAPAN, Band 8, Nr. 171 (P-293)[1608], 8. August 1984; & JP-A-59 67 445 (JIYUICHIROU OZAWA) 17-04-1984 <br> --- | 15,16 | **RECHERCHIERTE SACHGEBIETE (Int. Cl.4)** <br><br> G 01 N <br> G 01 M |
| A,D | FR-A-2 374 638 (B.M. POTTER) <br> * Seite 14, Zeilen 2-16,32-40 * <br> --- | 24,25 | |
| A | DE-B-1 299 141 (MOYAT et al.) <br> ----- | | |

Der vorliegende Recherchenbericht wurde für alle Patentansprüche erstellt

| Recherchenort | Abschlußdatum der Recherche | Prüfer |
|---|---|---|
| DEN HAAG | 09-09-1988 | ANTHONY R.G. |